# DEMANDE DE BREVET EUROPEEN

(11) **EP 1 093 844 A1**
(43) Date de publication de la demande: **25.04.2001**
(21) Numéro de dépôt: 00402370.1
(22) Date de dépôt: 28.08.2000
(51) Int. Cl.: B01F 17/56, B01F 17/54, A61K 7/48, A61K 7/06, A61K 7/02, A61K 9/107, B01F 17/00

(54) **Système émulsionnant pour une émulsion eau-dans-huile et ses utitilisations notamment dans le domaine cosmétique**

(30) Priorité: 21.10.1999 FR 9913150
(71) Demandeur: L'OREAL, 75008 Paris (FR)
(72) Inventeur: Lorant, Raluca, 94320 Thiais (FR)
(74) Mandataire: Rasson, Catherine

(57) **Abrégé**

La présente invention se rapporte à un système émulsionnant comprenant (1) un alkylpolyglycoside ayant un HLB inférieur à 7, et (2) un polydiméthylsiloxane oxyalkyléné.

La chaîne alkyle de l'alkylpolyglycoside comporte de préférence de 14 à 22 atomes de carbone et peut être notamment une chaîne linéaire insaturée ou une chaîne ramifiée, et plus particulièrement la chaîne oléyle ou isostéaryle.

L'invention se rapporte aussi aux émulsions eau-dans-huile, notamment cosmétiques, comprenant ce système, et aux utilisations de ces émulsions, notamment dans le domaine cosmétique.

Les émulsions de l'invention sont stables dans le temps et fraîches à l'application. Elles peuvent être destinées en particulier au soin de la peau, des lèvres, des ongles et des cheveux, au démaquillage et/ou au nettoyage de la peau, et/ou au maquillage de la peau et/ou des lèvres. Elles peuvent être plus particulièrement utilisées pour le traitement des peaux et/ou lèvres sèches et/ou sensibles.

## Description

La présente invention se rapporte à un système émulsionnant comprenant un alkylpolyglycoside ayant un HLB inférieur à 7 et un polydiméthylsiloxane oxyalkyléné. Elle se rapporte aussi aux émulsions eau-dans-huile, notamment cosmétiques, comprenant ce système et aux utilisations de ces émulsions, notamment dans le domaine cosmétique. Les émulsions de l'invention peuvent être destinées en particulier au soin de la peau, des lèvres, des ongles et des cheveux, au démaquillage et/ou au nettoyage de la peau, et/ou au maquillage de la peau et/ou des lèvres. Elles peuvent être plus particulièrement utilisées pour le traitement des peaux et/ou lèvres sèches et/ou peaux sensibles.

Dans le domaine cosmétique, il est courant d'utiliser des crèmes constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour le soin et la réparation des peaux sèches et déshydratées auxquelles elles apportent confort et protection grâce à la barrière lipidique qu'elles forment sur la peau. Elles conviennent aussi particulièrement bien pour le démaquillage de la peau et des lèvres grâce à la phase continue huileuse qui, en étant directement en contact avec le maquillage, exerce son action solvante sur celui-ci, d'où une meilleure efficacité et rapidité de démaquillage avec des émulsions E/H par rapport aux émulsions H/E.

Toutefois, malgré leur grande efficacité, les émulsions E/H sont minoritaires parmi les formes galéniques utilisées dans le domaine cosmétique car elles posent deux problèmes majeurs. Tout d'abord, ces émulsions ont l'inconvénient de manquer généralement d'agrément cosmétique, c'est-à-dire qu'elles sont grasses, lourdes, collantes et manquent de fraîcheur du fait de la phase externe huileuse. Elles sont en général difficiles à appliquer sur la peau, pénètrent difficilement et laissent sur la peau un film rémanent brillant et souvent collant.

Par ailleurs, les émulsions E/H présentent des problèmes de stabilité et il est souvent nécessaire, pour les stabiliser, d'utiliser un fort taux d'émulsionnants et/ou d'introduire une certaine quantité de facteurs de consistance, tels que les cires. Toutefois, ces ingrédients contribuent à accentuer les défauts cosmétiques (effet poisseux et gras) des émulsions E/H et il en résulte l'obtention de compositions souvent compactes et lourdes. De plus, si on augmente fortement la teneur en émulsionnant de ces émulsions pour remédier à leur instabilité, les émulsions obtenues peuvent se montrer irritantes vis-à-vis de certains types de peau, notamment les peaux sensibles.

En outre, dans le domaine du démaquillage, les émulsions E/H sont pratiquement absentes à cause de leurs caractéristiques sensorielles inadaptées aux gestes du démaquillage. De plus, du fait qu'elles laissent la peau grasse et brillante, il est indispensable de les éliminer par rinçage, mais ce rinçage est très difficile à réaliser avec un tonique aqueux.

Il subsiste donc le besoin d'une émulsion eau-dans-huile stable ne présentant pas les inconvénients rencontrés avec celles connues jusqu'à présent.

La demanderesse a trouvé de manière surprenante un système tensioactif comprenant un alkylpolyglycoside de HLB (Balance hydrophile lipophile) particulier et un polydiméthylsiloxane oxyalkyléné, permettait d'obtenir une émulsion eau-dans-huile ayant de bonnes propriétés cosmétiques (toucher léger, frais et riche à la fois) et une bonne stabilité dans le temps.

Aussi, la présente invention a pour objet un système émulsionnant comprenant (1) au moins un alkylpolyglycoside ayant un HLB inférieur à 7, et (2) au moins un polydiméthylsiloxane oxyalkyléné.

Les alkylpolyglycosides utilisés selon l'invention ont un HLB inférieur à 7 et de préférence inférieur ou égal à 5, tandis que les alkylpolyglycosides habituellement utilisés dans les compositions cosmétiques ont généralement un HLB supérieur à 10 et sont soit des détergents (HLB généralement supérieur à 13) soit des émulsionnants d'émulsion huile-dans-eau (HLB de 10 à 17 environ). Le HLB peut être calculé notamment par la méthode de Davies ou la méthode de Griffin.

Les alkylpolyglycosides utilisés selon la présente invention peuvent être plus particulièrement représentés par la formule générale (I) suivante :

R-O-(G)ₓ (I)

dans laquelle R représente un radical alkyle linéaire insaturé ou un radical alkyle ramifié, comportant de 14 à 24 atomes de carbone, G représente un sucre réduit comportant de 5 à 6 atomes de carbone, et x désigne une valeur allant de 1 à 15.

Des alkylpolyglycosides préférés selon la présente invention sont des composés de formule (I) dans laquelle R désigne plus particulièrement un radical alkyle comportant de 16 à 22 atomes de carbone, G désigne le glucose, le fructose ou le galactose, x est une valeur allant 1 à 4 et plus particulièrement de 1 à 2.

Selon l'invention, dans la formule (I), R est un radical alkyle linéaire insaturé (c'est-à-dire radical alkylène) ou un radical alkyle ramifié. Le radical alkyle insaturé peut comprendre une ou plusieurs insaturations éthyléniques, et en particulier une ou deux insaturations éthyléniques.

Selon un mode préféré de réalisation de l'invention, le radical R comporte 18 atomes de carbone et désigne notamment un radical oléyle (radical insaturé en C18) ou isostéaryle (radical saturé en C18), G désigne le glucose et x est une valeur allant de 1 à 2.

L'alkylpolyglycoside utilisé dans l'émulsion de l'invention est de préférence choisi dans le groupe comprenant l'isostéaryl-glucoside, l'oléyl-glucoside et leurs mélanges.

Selon un mode particulier de réalisation de l'invention, l'alkylpolyglycoside est introduit dans le système émulsionnant en mélange avec un alcool gras, et en particulier avec l'alcool gras comportant un groupe alkyl correspondant au groupe alkyl de l'alkylpolyglycoside, et par exemple l'alcool isostéarylique avec l'isostéaryl-glucoside et l'alcool oléylique avec l'oleyl-glucoside. Selon un mode préféré de réalisation de l'invention, on utilise dans le système émulsionnant de la présente invention, le mélange de l'alkylpolyglycoside tel que défini ci-dessus avec l'alcool gras correspondant sous forme d'une composition autoémulsionnante, comme décrit par exemple dans le document WO-A-92/06778.

Quand l'alkylpolyglycoside est en mélange avec l'alcool gras correspondant, ce dernier est présent en une quantité allant généralement de 60 à 90 % en poids et de préférence de 70 à 87 % en poids par rapport au poids du mélange d'alkylpolyglycoside et d'alcool gras.

La quantité d'alkylpolyglycoside(s) peut représenter par exemple de 15 à 60 % en poids de matière active et de préférence de 20 à 50 % en poids de matière active par rapport au poids total du mélange émulsionnant alkylpolyglycoside/ polydiméthylsiloxane oxyalkyléné.

Le polydiméthylsiloxane oxyalkyléné utilisé selon l'invention est plus particulièrement choisi parmi les composés de formule générale (II): 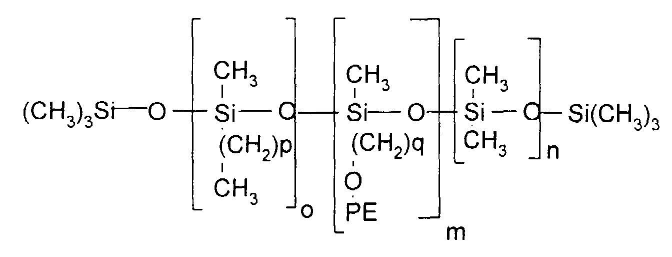 formule dans laquelle :
- PE représente (-C₂H₄O)ₓ(-C₃H₆O)_{y}-R, R étant choisi parmi un atome d'hydrogène et un radical alkyle de 1 à 4 atomes de carbone, x étant compris entre 10 et 100 et y compris entre 0 et 80;
- m est compris entre 1 et 25;
- n est compris entre 10 et 200;
- o est compris entre 0 et 100;
- p est compris entre 7 et 17;
- q est compris entre 0 et 4.

De préférence, on utilise un polydiméthylsiloxane oxyalkyléné répondant à la formule générale (II) dans laquelle q est égal à 3, o est compris entre 1 et 100, avec la condition que o n'est pas inférieur à m et que 3o est inférieur à n, et dans laquelle le poids moléculaire de PE est compris entre 250 et 2000 avec x et y choisis de manière à ce que leur rapport en poids x/y soit compris entre 100/0 et 20/80. On utilise avantageusement un polydiméthylsiloxane oxyalkyléné répondant à la formule générale (I) dans laquelle m égal à 5, n égal à 75, o compris entre 20 et 25, p égal à 15, q égal à 3, PE est complètement polyoxyéthyléné et a un poids moléculaire de 400.

Comme polydiméthylsiloxane oxyalkyléné pouvant entrer dans la composition selon l'invention, on peut citer les dimethicone copolyols et les alkyldiméthicone copolyols. Comme dimethicone copolyol, on peut citer par exemple le mélange de dimethicone copolyol, de cyclomethicone et d'eau (10/88/2), commercialisé par la société Dow Corning sous la dénomination DC3225C ou DC2-5225C, et le mélange de dimethicone copolyol et de cyclopentasiloxane (85/15) commercialisé sous la dénomination Abil EM-97 par la société Goldschmidt. Comme alkyldiméthicone copolyol, on peut citer notamment ceux ayant un radical alkyle comportant de 10 à 22 atomes de carbone, tel que le cétyl diméthicone copolyol comme le produit commercialisé sous la dénomination Abil EM-90 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning, et les mélanges de ces polydiméthylsiloxanes oxyalkylénés.

Selon un mode préféré de réalisation de l'invention, on utilise comme polydiméthylsiloxane oxyalkyléné, un alkyl diméthicone copolyol et plus particulièrement le cétyl diméthicone copolyol.

Le polydiméthylsiloxane oxyalkyléné peut représenter par exemple de 40 à 85 % en poids de matière active et de préférence de 50 à 80 % en poids de matière active par rapport au poids total du mélange émulsionnant alkylpolyglycoside/ polydiméthylsiloxane oxyalkyléné.

Le système émulsionnant selon l'invention est particulièrement approprié pour la préparation d'une émulsion eau-dans-huile.

L'invention a donc aussi pour objet l'utilisation du système émulsionnant tel que défini ci-dessus, pour la préparation d'une émulsion eau-dans-huile.

L'invention a encore pour objet une émulsion eau-dans-huile comportant une phase aqueuse dispersée dans une phase huileuse, caractérisée en ce qu'elle contient un système émulsionnant tel que défini ci-dessus.

Les émulsions obtenues selon l'invention présentent l'avantage de s'étaler facilement, et d'être absorbées rapidement et complètement dans la peau. En même temps qu'un toucher riche et nutritif, elles apportent une sensation de fraîcheur surprenante. Après pénétration du produit, la peau reste douce et mate. Par ailleurs, les émulsions de l'invention peuvent avoir une texture crème ou être fluides tout en ayant une bonne stabilité. Les émulsions fluides ont généralement une viscosité allant d'environ 0,2 à 3 Pa.s (2 à 30 poises) et de préférence de 0,6 à 2 Pa.s (6 à 20 poises), cette viscosité étant mesurée à environ 25°C à l'aide du viscosimètre "Rhéomat Metler" équipé d'un mobile 2 (pour les viscosités inférieures à 7 poises) ou d'un mobile 3 (pour les viscosités supérieures à 7 poises).

L'émulsion selon l'invention peut contenir une quantité de système émulsionnant allant de 0,5 à 20 % en poids de matière active, et de préférence de 1 à 10 % en poids de matière active par rapport au poids total de l'émulsion.

L'émulsion peut contenir, outre le système émulsionnant indiqué ci-dessus, un co-émulsionnant. De manière avantageuse, le co-émulsionnant peut être choisi dans le groupe comprenant les esters alkylés de polyol.

Comme ester alkylé de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Le co-émulsionnant s'il est présent peut être introduit en une quantité allant par exemple de 0.2 à 5 % et de préférence de 0.5 à 3 % en poids par rapport au poids total de l'émulsion.

La nature de la phase huileuse de l'émulsion selon l'invention n'est pas critique. La phase huileuse peut ainsi être constituée par tous les corps gras et notamment les huiles, classiquement utilisés dans le domaine cosmétique.

Parmi les huiles utilisables dans l'émulsion de l'invention, on peut citer par exemple les huiles végétales telles que l'huile de noyaux d'abricot et l'huile de soja ; les huiles minérales comme l'huile de vaseline ; les huiles de synthèse comme l'isohexadécane ; les huiles de silicone volatiles ou non volatiles et les huiles fluorées. Comme huiles de silicone volatiles, on peut citer notamment les polydiméthylsiloxanes cycliques ou cyclométhicones qui comportent d'environ 3 à 9 atomes de silicium et de préférence de 4 à 6 atomes de silicium, telles que le cyclohexadiméthylsiloxane ou cyclohexaméthicone et le cyclopentadiméthyl-siloxane ou cyclopentaméthicone, et les polydiméthylsiloxanes linéaires volatiles comportant d'environ 3 à 9 atomes de silicium. Les autres corps gras susceptibles d'être présents dans la phase huileuse peuvent être par exemple les acides gras, les alcools gras et les cires telles que la vaseline ou la cire d'abeille.

Quand l'émulsion est utilisée comme produit de démaquillage de la peau et/ou des yeux, elle contient plus particulièrement des huiles démaquillantes et notamment celles choisies parmi les esters d'acide gras comportant au moins 12 atomes de carbone. Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou di-esters. Les huiles démaquillantes peuvent être notamment choisies dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

La quantité de phase huileuse dans l'émulsion de l'invention peut aller par exemple de 5 à 50 % et de préférence de 10 à 40 % en poids par rapport au poids total de l'émulsion.

La phase aqueuse de l'émulsion peut représenter par exemple de 50 à 95 % et de préférence de 60 à 90 % en poids par rapport au poids total de l'émulsion.

L'émulsion de l'invention peut constituer notamment une composition cosmétique ou dermatologique, destinée à une application sur la peau, les cheveux et/ou les muqueuses. Elle contient alors avantageusement un milieu physiologiquement acceptable, c'est-à-dire compatible avec la peau, les muqueuses (lèvres) et/ou les cheveux.

De façon connue, l'émulsion de l'invention peut contenir également des adjuvants habituels dans le domaine cosmétique, tels que les actifs, les conservateurs, les antioxydants, les agents complexants, les solvants, les parfums, les charges, les filtres, les bactéricides, les absorbeurs d'odeur, les matières colorantes (pigments ou colorants solubles) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase huileuse, dans la phase aqueuse et/ou dans les vésicules lipidiques.

Comme charges qui peuvent être utilisées dans l'émulsion de l'invention, on peut citer par exemple, outre les pigments, les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ méthacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges.

De manière préférée, on utilise comme charge les microsphères EXPANCEL, qui sont des particules de terpolymère expansé de chlorure de vinylidène, d'acrylonitrile et de méthacrylate, et notamment celles vendues sous les références 551 ayant une granulométrie allant d'environ 10 à 100 µm (551 DE 12, 551 DE 20, 551 DE 50, 551DE 80) et celles vendues sous la référence EL 23 (granulométrie d'environ 18 µm).

Lorsque l'émulsion de l'invention contient des charges, la quantité de charge(s) peut aller par exemple de 0,01 à 15 % et de préférence de 0,1 à 5 % en poids par rapport au poids total de l'émulsion.

L'émulsion selon l'invention peut en outre contenir un ou plusieurs sels, et notamment un sel de magnésium tel que le sulfate de magnésium. La quantité de sel(s) peut aller par exemple de 0,1 à 5 % et de préférence de 0,5 à 1 % en poids par rapport au poids total de l'émulsion.

L'émulsion selon l'invention trouve son application dans un grand nombre de traitements, notamment cosmétiques, de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres. Elle peut être destinée aussi au traitement des peaux sèches et/ou des lèvres sèches et/ou peaux sensibles.

L'émulsion selon l'invention peut par exemple être utilisée comme produit de soin, de démaquillage et/ou de nettoyage pour le visage sous forme de crèmes ou de laits ou comme produits de maquillage (peau et lèvres), et par exemple fonds de teint, par incorporation de charges ou de colorants.

Aussi, l'invention a encore pour objet l'utilisation cosmétique de l'émulsion telle que définie ci-dessus pour le traitement, la protection, le soin, le démaquillage et/ou le nettoyage de la peau, des lèvres et/ou des cheveux, et/ou pour le maquillage de la peau et/ou des lèvres.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une émulsion telle que définie ci-dessus.

L'invention a aussi pour objet l'utilisation de l'émulsion telle que définie ci-dessus pour la fabrication d'une composition destinée au soin des peaux sèches et/ou des lèvres sèches et/ou des peaux sensibles.

Les exemples ci-après d'émulsions selon l'invention sont donnés à titre d'illustration et sans caractère limitatif. Les quantités y sont données en % en poids, sauf mention contraire.

### Exemple 1 : Fluide hydratant pour tous types de peaux

| *Phase huileuse :* | |
|---|---|
| Isostéaryl-glucoside/alcool isostéarylique (15/85) | 5 % |
| (soit 0,75 % de matière active d'alkyl-polyglycoside) | |
| Cetyl dimethicone copolyol | 1 % |
| Isohexadecane | 15 % |
| Cyclohexaméthicone | 10 % |

| *Charge* | |
|---|---|
| Expancel 551 | 1 % |

| *Phase aqueuse* | |
|---|---|
| Glycérine | 5 % |
| Sulfate de magnésium | 0,5 % |
| Conservateurs | 0,4 % |
| Eau déminéralisée | qsp 100 % |

Mode opératoire : On disperse la charge dans la phase huileuse, puis on émulsionne en dispersant sous agitation la phase aqueuse dans le mélange obtenu.

On obtient ainsi un fluide, très doux à l'application, riche et frais à la fois, qui n'est pas gras et qui pénètre rapidement dans la peau qu'il laisse douce, mate et souple.

### Exemple 2 : Crème de soin nourrissante pour peaux sèches et sensibles

| *Phase huileuse :* | |
|---|---|
| Isostéaryl-glucoside/alcool isostéarylique (15/85) | 3 % |
| (soit 0,45 % de matière active d'alkyl-polyglycoside) | |
| Cetyl dimethicone copolyol | 1 % |
| Polyglyceryl 4-isostearate | 0,5 % |
| Cire d'abeille | 1 % |
| Cyclohexaméthicone | 8 % |

| *Phase aqueuse* | |
|---|---|
| Glycérine | 5 % |
| Sulfate de magnésium | 0,5 % |
| Conservateurs | 0,4 % |
| Eau déminéralisée | qsp 100 % |

Mode opératoire : On chauffe séparément à 75°C les phases huileuse et aqueuse, puis on émulsionne en dispersant sous agitation la phase aqueuse dans la phase huileuse.

On obtient ainsi une crème onctueuse, douce et riche à l'application. Elle pénètre facilement en apportant immédiatement un effet nutritif apaisant.

### Exemple 3 : Lait démaquillant pour peaux sèches

| *Phase huileuse :* | |
|---|---|
| Oleyl-glucoside/alcool oléylique (16,5/83,5) | 3 % |
| (soit 0,495 % de matière active d'alkyl-polyglycoside) | |
| Dimethicone copolyol/cyclopentasiloxane (85/15) (Abil EM97) | 2 % |
| (soit 1,7 % de matière active de polydiméthylsiloxane oxyalkyléné) | |
| Huile de vaseline | 10 % |
| Cyclopentaméthicone | 10 % |
| Palmitate d'octyle | 10 % |

| *Charge* | |
|---|---|
| Expancel 551 | 0,5 % |

| *Phase aqueuse* | |
|---|---|
| Glycérine | 5 % |
| Sulfate de magnésium | 0,5 % |
| Conservateurs | 0,4 % |
| Eau déminéralisée | qsp 100 % |

Mode opératoire : On disperse la charge dans la phase huileuse, puis on émulsionne en dispersant sous agitation la phase aqueuse dans le mélange obtenu.

On obtient ainsi un lait particulièrement agréable lors de l'application sur la peau, présentant de très bonnes propriétés démaquillantes. Il est très onctueux à l'application et élimine en douceur le maquillage et les impuretés sans agresser ni irriter la peau.

Après élimination, il laisse la peau douce, mate et fraîche.

### Exemple 4 : Fond de teint pour peaux sèches

| *Phase huileuse :* | |
|---|---|
| Isostéaryl-glucoside/alcool isostéarylique (15/85) | 4 % |
| (soit 0,6 % de matière active d'alkyl-polyglycoside) | |
| Cetyl dimethicone copolyol | 2 % |
| Huile de soja | 2 % |
| Cyclohexaméthicone | 18 % |

| *Charges* | |
|---|---|
| Pigments | |

| *Phase aqueuse* | |
|---|---|
| Glycérine | 5 % |
| Sulfate de magnésium | 0,5 % |
| Conservateurs | 0,4 % |
| Eau déminéralisée | qsp 100 % |

Mode opératoire : On disperse les charges dans la phase huileuse, puis on émulsionne en dispersant sous agitation la phase aqueuse dans la phase huileuse.

On obtient ainsi un fond de teint qui a une texture souple et qui est très doux à l'application sur la peau. En outre, il est facile à étaler et donne un résultat de maquillage homogène, mat et naturel.

## Revendications

1. Système émulsionnant comprenant (1) au moins un alkylpolyglycoside ayant un HLB inférieur à 7, et (2) au moins un polydiméthylsiloxane oxyalkyléné.

2. Système selon la revendication 1, caractérisé en ce que l'alkylpolyglycoside a un HLB inférieur ou égal à 5.

3. Système selon la revendication 1 ou 2, caractérisée par le fait que l'alkylpolyglycoside est représenté par la formule générale (I) suivante :
R-O-(G)ₓ (I)
dans laquelle R représente un radical alkyle linéaire insaturé ou un radical alkyle ramifié, comportant de 14 à 24 atomes de carbone, G représente un sucre réduit comportant de 5 à 6 atomes de carbone, et x désigne une valeur allant de 1 à 15.

4. Système selon la revendication 3, caractérisé par le fait que dans la formule (I), R désigne plus particulièrement un radical alkyle comportant de 16 à 22 atomes de carbone, G désigne le glucose, le fructose ou le galactose, x est une valeur allant 1 à 4 et plus particulièrement de 1 à 2.

5. Système selon l'une quelconque des revendications précédentes, caractérisé en ce qu'il contient un alcool gras comportant un groupe alkyle correspondant au radical alkyl de l'alkylpolyglycoside.

6. Système selon l'une quelconque des revendications précédentes, caractérisée en ce que l'alkylpolyglycoside est choisi dans le groupe comprenant l'isostéaryl-glucoside, l'oléyl-glucoside et leurs mélanges.

7. Système selon l'une quelconque des revendications précédentes, caractérisé par le fait que le polydiméthylsiloxane oxyalkyléné est un composé de formule générale (II): 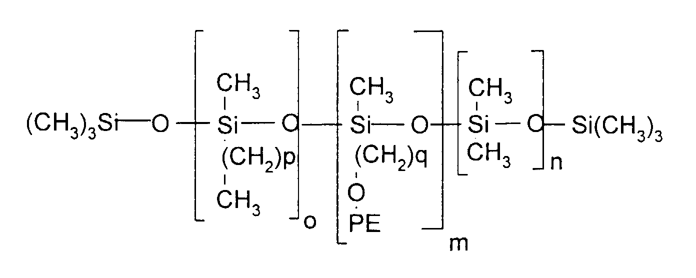 formule dans laquelle :
- PE représente (-C₂H₄O)ₓ(-C₃H₆O)_{y}-R, R étant choisi parmi un atome d'hydrogène et un radical alkyle de 1 à 4 atomes de carbone, x étant compris entre 10 et 100 et y compris entre 0 et 80;
- m est compris entre 1 et 25;
- n est compris entre 10 et 200;
- o est compris entre 0 et 100;
- p est compris entre 7 et 17;
- q est compris entre 0 et 4.

8. Système selon la revendication précédente, caractérisé par le fait que le polydiméthylsiloxane oxyalkyléné est un alkyl diméthicone copolyol ayant un radical alkyle comportant de 10 à 22 atomes de carbone.

9. Système selon l'une quelconque des revendications précédentes, caractérisé par le fait qu'il comprend de 15 à 60 % en poids de matière active d'au moins un alkylpolyglycoside ayant un HLB inférieur à 7, et de 40 à 85 % en poids de matière active d'au moins un polydiméthylsiloxane oxyalkyléné.

10. Emulsion eau-dans-huile comportant une phase aqueuse dispersée dans une phase huileuse, caractérisée en ce qu'elle comprend un système émulsionnant selon l'une quelconque des revendications précédentes.

11. Emulsion selon la revendication précédente, caractérisée en ce que le système émulsionnant est présent en une quantité allant de 0,5 à 20 % en poids de matière active par rapport au poids total de l'émulsion.

12. Emulsion selon la revendication 10 ou 11, caractérisée en ce qu'elle contient un co-émulsionnant choisi dans le groupe comprenant les esters alkylés de polyol.

13. Emulsion selon l'une quelconque des revendications 10 à 12, caractérisée en ce que la quantité de phase huileuse va de 5 à 50 % en poids par rapport au poids total de l'émulsion.

14. Emulsion selon l'une quelconque des revendications 10 à 13, caractérisée en ce qu'elle comprend au moins une charge.

15. Emulsion selon l'une quelconque des revendications 10 à 14, caractérisée en ce qu'elle constitue une composition cosmétique.

16. Utilisation cosmétique de l'émulsion selon l'une quelconque des revendications 10 à 15 pour le traitement, la protection, le soin, le démaquillage et/ou le nettoyage de la peau, des lèvres et/ou les cheveux, et/ou pour le maquillage de la peau et/ou des lèvres.

17. Procédé de traitement cosmétique de la peau, des cheveux, et/ou des lèvres, caractérisé par le fait qu'on applique sur la peau, les cheveux et/ou les lèvres, une émulsion selon l'une quelconque des revendications 10 à 15.

18. Utilisation de l'émulsion selon l'une quelconque des revendications 10 à 15 pour la fabrication d'une composition destinée au soin des peaux sèches et/ou des lèvres sèches et/ou des peaux sensibles.

19. Utilisation du système émulsionnant selon l'une quelconque des revendications 1 à 9, pour la préparation d'une émulsion eau-dans-huile.
